# EUROPEAN PATENT APPLICATION

(11) **EP 4 650 778 A1**
(43) Date of publication of application: **19.11.2025**
(21) Application number: 24741246.3
(22) Date of filing: 09.01.2024
(51) Int. Cl.: G01N 33/574, G01N 33/68, A61K 45/00, A61P 35/00, A61P 35/02, C07K 14/47, C07K 16/24, C12Q 1/6886

(54) **USE OF ABCG1 PHOSPHORYLATION SITE AS TUMOR BIOMARKER**

(30) Priority: 10.01.2023 CN 202310033024
(71) Applicant: Fudan University Shanghai Cancer Center, Xuhui, Shanghai 200032 (CN)
(72) Inventor: YANG, Gong, Shanghai 200032 (CN)
(74) Representative: Alatis
(86) International application number: PCT/CN2024/071371
(87) International publication number: WO 2024/149249

(57) **Abstract**

Use of a reagent and/or device for detecting a human ABCGI phosphorylation site in the preparation of a reagent for assessing the risk of tumor occurrence, diagnosing tumors or assessing tumor prognosis, and a kit; and use of a reagent for inhibiting a human ABCG1 phosphorylation site in the preparation of a drug for preventing and/or treating tumors, and a kit. The human ABCG1 phosphorylation site is selected from one or more of Y97, T99, S120, S125, T272, S277, S441 and Y655. A human ABCG1 protein phosphorylated antigen peptide, an antibody, and the use thereof and a kit. Any one or more of the ABCGI phosphorylation sites has great clinical medical significance for tumor occurrence risk assessment, diagnosis, treatment, prognostic assessment, etc.

## Description

### TECHNICAL FIELD

The present disclosure belongs to the technical field of molecular diagnostics, and specifically relates to use of an ABCG1 phosphorylation site as a biomarker in diagnosis and prognostic assessment of a tumor.

### BACKGROUND

The ATP-binding cassette (ABC) transporter superfamily consists of a class of membrane proteins, and ATP-binding cassette subfamily G member 1 (ABCG1) is a member of the ABCG subfamily. Early studies showed that ABCG1 is primarily responsible for the transport of cholesterol and phospholipids in macrophages and may also regulate the lipid homeostasis in some cells.

In recent years, various studies on the association of ABCG1 with tumors have been carried out. Roundhill et al. (Roundhill E.A. et al., Cancer Lett, 453 (2019) 142-157) found that ABCG1 is the only gene up-regulated in the self-renewing drug-resistant cell line HOS-EC50.SR, suggesting that targeting ABCG1 may eliminate the drug-resistant self-renewing cells, osteosarcoma cancer stem-like cells (OST-CSCs), thereby improving the survival outcomes for osteosarcoma patients. Additionally, ABCG1 is critically important for the survival of malignant glioma cells, and thus targeting ABCG1 may be beneficial for the treatment of malignant gliomas (Chen Y.H. et al., Oncotarget, 7 (2016) 23416-23424). The deletion of ABCG1 has been shown to inhibit tumor growth by regulating macrophage behaviour within the tumor. ABCG1 was also found to be involved in the induction of cisplatin resistance and tumor metastasis of lung adenocarcinoma cells following chemotherapy. Molecules such as ABCG1 are expressed at a higher level in normal CD34+/CD38- bone marrow cells than in acute myeloid leukemia CD34+/CD38- cells, indicating differences in cholesterol metabolism levels. Liver X receptor (LXR) inhibitors can suppress the differentiation of breast cancer cells MCF-7 and induce apoptosis in these cells, while LXR activation can significantly promote the expression of ABCG1 and the cholesterol efflux by cells. Saracatinib can up-regulate the expression of ABCG1, thereby diminishing the antitumor efficacy of oxaliplatin. Li Hongtao et al. (Li Hongtao et al., Chinese Journal of Experimental Surgery, 37 (2020) 721-723) have discovered that miR-519 may regulate the sensitivity of breast cancer to gemcitabine by targeting ABCG1.

In terms of tumor metastasis, only ABCG1 was found to possibly mediate the metastasis and cisplatin resistance of lung adenocarcinoma along with molecules such as Slug (Zhan J. et al., Theranostics, 9 (2019) 2084-2099). In studies related to ovarian cancer, ABCG1 and related molecules were up-regulated in progesterone-treated normal ovarian epithelial cells to regulate the balance of cholesterol and lipids in the cells, which may provide insights for the prevention and treatment of ovarian cancer (Paucarmayta A. et al., Biomedicines, 8 (2020)). ABCG1 is highly expressed in high-grade serous ovarian cancer and is closely related to survival (Wilcox C. B. et al., BMC Cancer, 7 (2007) 223). *Scutellaria baicalensis* (SB) extract can inhibit molecules including ABCG1 to enhance the killing effects of platinum-based drugs for ovarian cancer cells (Elsnerova K. et al., Oncology Reports, 35 (2016) 2159-2170). A recent study revealed that a combination of progesterone-calcitriol with platinum-based drugs could down-regulate the expression of molecules including ABCG1 to restrain the drug efflux, thereby boosting the killing of the platinum-based drugs for ovarian cancer cells (Hussain I. et al., Journal of Cellular Biochemistry, 119 (2018) 7515-7524). Collectively, ABCG1 is closely associated with the occurrence, metastasis, and drug resistance of tumors.

There have been reports on the phosphorylation modification and functional regulation for members of the ABCA, ABCB, and ABCC subfamilies in the ABC transporter superfamily. Among the ABCD subfamily, only ABCD1 and ABCD3 undergo tyrosine phosphorylation. In the ABCG subfamily, the ABCG2 (T362) molecule is currently known to include a definite phosphorylation site. According to a series of studies by Nagelin et al., ABCG1 in macrophages can be degraded by 12/15-lipoxygenase (12/15-LO) through JNK2 and p38-mediated N-terminal serine phosphorylation to cause the cholesterol accumulation and atherosclerosis in macrophages (Nagelin M.H. et al., Arteriosclerosis, Thrombosis, and Vascular Biology, 28 (2008) 1811-1819). In these studies, S27A, S28A, S43A, S45A, S80A, and S85A mutants were constructed with the ABCG1 isoform (ORF638), but degraded by 12/15-LO. Accordingly, S65A, S70A, S119A, S141A, and S168A mutants were constructed, and these mutants could resist the phosphorylation and degradation of ABCG1 induced by 12/15-LO. This suggests that these serine sites at the N-terminus of ABCG1 are likely phosphorylated and mediate the degradation of ABCG1 (Nagelin M.H. et al., Journal of Biological Chemistry, 284 (2009) 31303-31314). However, the specific functional phosphorylation sites have not yet been identified. Ogura et al. found that the degradation of ABCG1 could be related to the ubiquitin proteasome pathway (Ogura M. et al., Arteriosclerosis, Thrombosis, and Vascular Biology, 31 (2011) 1980-1987), but there are no in-depth and clear reports accordingly. Gelissen et al. used protein kinase A (PKA) inhibitors to investigate the functions associated with cholesterol regulation and the stability of the ABCG1 protein based on potential phosphorylation sites predicted for the two ABCG1 isoforms of ABCG1(+12) and ABCG1(-12). S389 in ABCG1(+12) is very important for this isoform to regulate the cholesterol efflux and protein stability, and was regulated by a PKA inhibitor, making phosphorylation possible. The S377 site corresponding to ABCG1(-12) was not affected by the PKA inhibitor, indicating that there may be no phosphorylation of S377 in ABCG1(-12) (Gelissen I.C. et al., Journal of Lipid Research, 53 (2012) 2133-2140). However, the specific phosphorylation sites of ABCG1 remain undetermined in this study. Recently, Watanabe discovered that the phosphorylation of ABCG1 by the protein kinase C (PKC) could enhance the stability of ABCG1 and suppress the degradation of ABCG1, thereby facilitating the cholesterol efflux (Watanabe T. et al., Journal of Biochemistry, 166 (2019) 309-315). However, the specific phosphorylation sites of ABCG1 by PKC remain unclear.

In summary, it is of great significance to explore the specific phosphorylation sites of ABCG1 and utilize these phosphorylation sites for tumor risk assessment, diagnosis, treatment, and prognostic assessment.

### SUMMARY

To address the aforementioned problems, the present disclosure has discovered and validated use of an ABCG1 phosphorylation site and an antibody thereof in diagnosis, treatment, and prognosis of a tumor. It is specifically as follows:
A first aspect of the present disclosure provides use of a reagent and/or device for detecting a human ABCG1 phosphorylation site in preparation of a reagent for risk assessment, diagnosis, or prognostic assessment of a tumor.

In some embodiments, the human ABCG1 phosphorylation site is selected from the group consisting of Y97, T99, S120, S125, T272, S277, S441, and Y655.

In some embodiments, the tumor is selected from the group consisting of osteosarcoma, glioma, lung cancer, leukemia, breast cancer, and ovarian cancer.

A second aspect of the present disclosure provides use of a reagent for inhibiting a human ABCG1 phosphorylation site in preparation of a drug for preventing and/or treating a tumor.

In some embodiments, the human ABCG1 phosphorylation site is selected from the group consisting of Y97, T99, S120, S125, T272, S277, S441, and Y655.

In some embodiments, the tumor is selected from the group consisting of osteosarcoma, glioma, lung cancer, leukemia, breast cancer, and ovarian cancer.

A third aspect of the present disclosure provides a human ABCG1 protein phosphorylation-specific antigenic peptide, where the human ABCG1 protein phosphorylation-specific antigenic peptide is any one selected from the group consisting of amino acid sequences of SEQ ID NOs: 1-8.

A fourth aspect of the present disclosure provides a human ABCG1 protein phosphorylation site-specific antibody, where the human ABCG1 protein phosphorylation site-specific antibody is produced by immunizing an animal with the human ABCG1 protein phosphorylation-specific antigenic peptide described in the third aspect of the present disclosure.

A fifth aspect of the present disclosure provides use of the human ABCG1 protein phosphorylation-specific antigenic peptide described in the third aspect of the present disclosure in preparation of a formulation for detecting an ABCG1 protein phosphorylation site.

A sixth aspect of the present disclosure provides use of the human ABCG1 protein phosphorylation site-specific antibody described in the fourth aspect of the present disclosure in preparation of a formulation for detecting an ABCG1 protein phosphorylation site.

In some embodiments, the ABCG1 protein phosphorylation site is selected from the group consisting of Y97, T99, S120, S125, T272, S277, S441, and Y655.

A seventh aspect of the present disclosure provides a kit for risk assessment, diagnosis, prevention, treatment, or prognostic assessment of a tumor, including the reagent for detecting a human ABCG1 phosphorylation site described in the first aspect of the present disclosure, the reagent for inhibiting/promoting a human ABCG1 phosphorylation site described in the second aspect of the present disclosure, or the human ABCG1 protein phosphorylation site-specific antibody described in the fourth aspect of the present disclosure.

Compared with the prior art, embodiments of the present disclosure have the following beneficial effects.

The present disclosure has confirmed that, in the ABCG1 protein, at least 8 amino acid sites, namely, Y97, T99, S120, S125, T272, S277, S441, and Y655, are phosphorylated. It is of significant clinical medical significance to use any one or more of these sites for risk assessment, diagnosis, treatment, prognostic assessment, etc. of one or more tumors (including, but not limited to, ovarian cancer).

The present disclosure also provides a human ABCG1 protein phosphorylation-specific antigenic peptide, which can be used to prepare a human ABCG1 protein phosphorylation site-specific antibody. The resulting antibody can be used for the detection and functional research of ABCG1 protein phosphorylation sites.

### BRIEF DESCRIPTION OF THE DRAWINGS

Other features, objectives, and advantages of the present disclosure will become more apparent by reading the detailed description of non-limiting embodiments with reference to the following accompanying drawings.

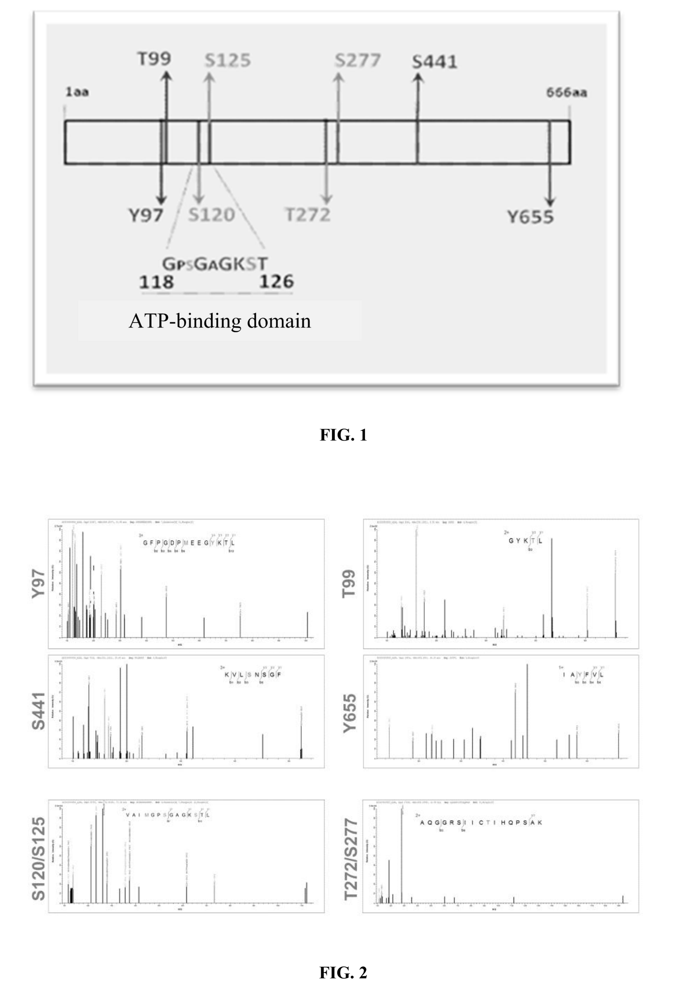

FIG. 1 shows the ABCG1 phosphorylation sites acquired by mass spectrometry, where S120 and S125 may be phosphorylated at only one site or both; similarly, T272 and S277 may be phosphorylated at only one site or both.;
FIG. 2 shows the secondary mass spectrometry spectra determined for ABCG1 phosphorylation sites;

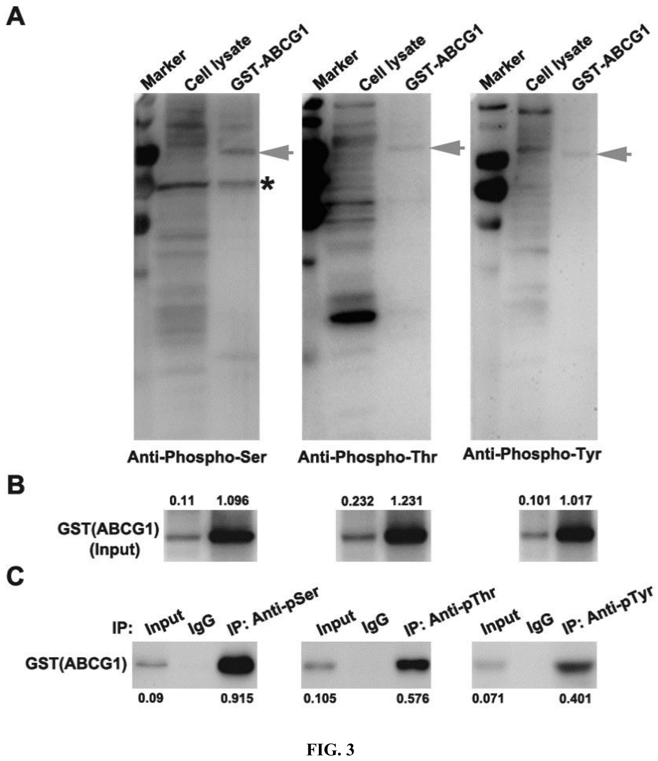

FIG. 3 shows the detection results of anti-ABCG1 phosphorylation antibodies, where * means that the appearance of non-target bands in the GST-ABCG1 sample detected by an anti-phosphorylated Ser antibody may be due to trace amounts of other serine-phosphorylated proteins in the purified product; and the GST antibody comes from Cell Signaling Technology (CST) #2622S; and pSer/pThr/pTyr antibodies come from Abcam, #ab9332/ab9337/ab10321;

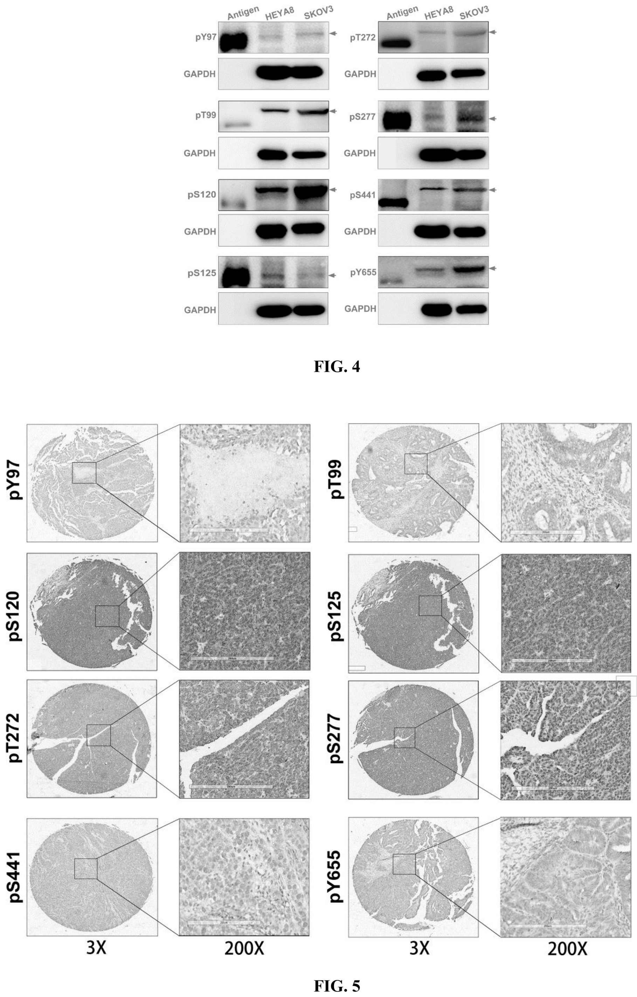

FIG. 4 shows the detection results of ABCG1 phosphorylation in ovarian cancer cell lines SKOV3 and HEYA8, where the ABCG1 phosphorylation site-specific antibody is adopted as a primary antibody; the bovine serum albumin (BSA)-conjugated phosphorylated polypeptide fragment is adopted as a control antigen; GAPDH is a loading control for the cell samples; and the antibody comes from Cell Signaling Technology, catalog No. #5174; and
FIG. 5 shows the immunohistochemistry (IHC) detection results of ABCG1 phosphorylation in ovarian cancer tissues, where the ABCG1 phosphorylation site-specific antibody is adopted as a primary antibody and horseradish peroxidase (HRP)-conjugated goat anti-rabbit IgG is adopted as a secondary antibody; and 3,3'-diaminobenzidine (DAB) is adopted as a chromogenic reagent.

### DETAILED DESCRIPTION OF THE EMBODIMENTS

To make the objectives, technical solutions, and advantages of the embodiments of the present disclosure clear, the technical solutions in the embodiments of the present disclosure are clearly and completely described below with reference to the accompanying drawings in the examples of the present disclosure. Apparently, the described examples are merely some rather than all of the embodiments of the present disclosure. Based on the described embodiments of the present disclosure, all other embodiments obtained by those of ordinary skill in the art without creative efforts shall fall within the protection scope of the present disclosure.

### Example 1 Experimental materials and methods

### 1.1 GST pull-down assay in eukaryotic cells

Cells were collected from a 10 cm dish and lysed to determine the protein concentration. Next, 500 µg of protein was taken, and the volume was adjusted to 1mL with cell lysis buffer. Then, 20 µL of Glutathione Sepharose 4B was added to the cell lysate. Incubation was allowed for 2 h under rotating in a 4°C shaker. The mixture was then centrifuged in at 4°C at 500 g for 5 min, and the supernatant was discarded. One milliliter of cell lysis buffer was added, and inverting was repeated for washing. Centrifugation was conducted for 5 min in a 4°C centrifuge at 500 g. The steps (4) and (5) were repeated twice. Then, 20 µL to 40 µL of a 2×Sodium Dodecyl Sulfate (SDS) loading buffer was added to the pellet. A resulting sample was boiled in a 100°C metal bath for 2 min to 4 min. The supernatant was collected for sodium dodecyl sulfate-polyacrylamide gel electrophoresis (SDS-PAGE). The band corresponding to the molecular weight of ABCG1 was cut.

### 1.2 Mass spectrometry analysis of ABCG1 protein phosphorylation

Gels cut from the GST pull-down product were directly sent to APTBIO for protein modification analysis by mass spectrometry. Basic steps were as follows:

The protein analysis was performed using Thermo Q Exactive Plus Orbitrap LC-MS/MS as a mass spectrometer. Peptide fragments were filtered with 1% false discovery rate (FDR) and 1 unique peptide for quality control.

Tandem mass spectrometry data was analyzed by Mascot for database searching, with trypsin digestion set as the enzyme. Database search parameters included a fragment ion mass tolerance of 0.05 Da, a precursor ion mass tolerance of 7 ppm, and a maximum missed cleavage count of 4.

Fixed modifications comprised carbamidomethylation (57.02). Variable modifications: oxidation (M): 15.99, phosphorylation (STY): 79.97, and acetylation (protein N-term): 42.01. The present disclosure focused on phosphorylation modifications, and finally identified the corresponding secondary mass spectrometry spectra from phosphorylated peptide fragments.

### 1.3 Preparation of anti-ABCG1 phosphorylation antibodies

**A.** Synthesis of peptide fragments including phosphorylated and non-phosphorylated peptide fragments, as shown in Table 1.

**Table 1 Synthesis of phosphorylated and non-phosphorylated peptide fragments**

| **Phosphorylated peptide fragments** | **Non-phosphorylated peptide fragments** |
|---|---|
| *Cys+PEGPWWRKKGY(p)KT (Y97) (SEQ ID NO: 1) | Cys+PEGPWWRKKGYKT (Y97) (SEQ ID NO: 9) |
| RKKGYKT(p)LLKGISGK+Cys(T99) (SEQ ID NO: 2) | RKKGYKTLLKGISGK+Cys(T99) (SEQ ID NO: 10) |
| Cys+MGPS(p)GAGKSTLMN(S120) (SEQ ID NO: 3) | Cys+MGPSGAGKSTLMN(S120) (SEQ ID NO: 11) |
| Cys+MGPSGAGKS(p)TLMN(S125) (SEQ ID NO: 4) | Cys+MGPSGAGKSTLMN(S125) (SEQ ID NO: 12) |
| Cys+QGGRSIICT(p)IHQP(T272) (SEQ ID NO: 5) | Cys+QGGRSIICTIHQP(T272) (SEQ ID NO: 13) |
| HQPS(p)AKLFELFDQ+Cys(S277) (SEQ ID NO: 6) | HQPSAKLFELFDQ+Cys(S277) (SEQ ID NO: 14) |
| Cys+GNEAKKVLS(p)NSG (S441) (SEQ ID NO: 7) | Cys+GNEAKKVLSNSG (S441) (SEQ ID NO: 15) |
| AY(p)FVLRYKIRAER+Cys(Y655) (SEQ ID NO: 8) | AYFVLRYKIRAER+Cys(Y655) (SEQ ID NO: 16) |

| | |
|---|---|
| *: The addition of an amino acid Cys at an N-terminus or C-terminus of a peptide fragment enables the subsequent conjugation of the peptide fragment with macromolecules such as keyhole limpet hemocyanin (KLH) and BSA. | |

### B. Animal immunization

New Zealand white rabbits (female, > 2.5 kg, Shandong Ailaike Biotechnology Co., Ltd., animal experiment license No.: SYXK (Lu) 20190018) were used. The rabbit was secured on a platform. Hindquarters of the rabbit were pressed by one hand and the head of the rabbit was pressed by the other hand to keep the rabbit still, with the cervicodorsal region exposed as much as possible. Each immunization point and its surroundings on the cervicodorsal region were disinfected with 75% alcohol. The skin was lifted by one hand, and an immunization needle was held by the other hand, inserted along the direction of the thumb of the hand lifting the skin to a depth of about 0.5 cm for injection. There were 5 injection points in total. The priming was conducted with a mixture of a phosphorylated polypeptide (concentration: 1 mg/mL) and a Freund's complete adjuvant (F5881, SIGMA). The boosting was conducted thrice at different sites with a mixture of a phosphorylated polypeptide (concentration 1 mg/mL) and a Freund's incomplete adjuvant (F5506, SIGMA). Immunization time and dose details were shown in Table 2.

**Table 2 Animal immunization times, doses, and protocols**

| Immunization time | Mass (mg/rabbit) | Volume (µL) | Adjuvant (µL) | Total volume (µL) | Immunization protocol |
|---|---|---|---|---|---|
| First immunization (Day 1) | 0.5mg | 500 | 500 | 1000 | Intradermal injection at 2 points and subcutaneous injection at 3 points |
| Second immunization (Day 15) | 0.5mg | 500 | 500 | 1000 | |
| Third immunization (Day 25) | 0.5mg | 500 | 500 | 1000 | |
| Fourth immunization (Day 30) | 0.5mg | 500 | 500 | 1000 | |
| Fifth immunization (Day 37) | 0.5mg | 500 | 500 | 1000 | |
| Sixth immunization (Day 44) | 0.5mg | 500 | 500 | 1000 | |

### C. Detection of antibody titer by enzyme-linked immunosorbent assay (ELISA)

C-1. Coating: The required volume of antigen and coating solution were calculated, with the protein coating concentration set at 1 µg/mL to 2 µg/mL and the polypeptide coating concentration set at 1 µg/mL to 5 µg/mL.

C-2. Blocking: The coated microplate was taken out from a 37°C incubator or a 4°C refrigerator, the residual coating solution in the coated microplate was discarded into a water tank, and 300 µL of 5% milk was then added to each well. The microplate was covered and incubated in a 37°C incubator for 1 h. When the blocking was conducted after 4 P.M., the incubation was conducted overnight in a 4°C refrigerator.

C-3. Washing: The coated and blocked microplate was taken out from the 37°C incubator or the 4°C refrigerator, the residual blocking solution was discarded into a water tank, and washing was conducted three times with phosphate-buffered saline with Tween (PBST). Plates were arranged according to item numbers and sequence numbers, then packed into bags, and stored at -20°C for later use. These plates had a shelf life of 2 months, and should be discarded if stored for more than 2 months. When immediate addition was required, the primary antibody was directly added to the plate, and then the plate was incubated without the need for storage at -20°C. Primary antibody incubation: According to experimental requirements, the primary antibody was added at 100 µL/well to a plate. The plate was covered, incubated in a 37°C incubator for 60 min, then taken out, spin-dried to remove the primary antibody, washed three times with PBST, and pat-dried on absorbent paper.

C-4. Secondary antibody incubation: According to experimental requirements, the corresponding secondary antibody was added at 100 µL/well to the plate. The plate was covered, incubated in a 37°C incubator for 30 min, then taken out, spin-dried to remove the secondary antibody, washed three times with PBST, and pat-dried on absorbent paper.

C-5. Color development with 3,3',5,5'-tetramethylbenzidine (TMB): A prepared TMB chromogen solution was poured into a clean dispensing trough lined with a polyethylene (PE) glove, and added through a multichannel pipette at 100 µL/well to a microplate. The microplate was then covered and placed in an incubator for 5 min to 10 min, and the color development was observed.

C-6. Termination: The microplate reader was turned on and preheated for 1 min. A microplate was taken out from an incubator, and 2 M HCl was added at 50 µL/well to the microplate.

C-7. Reading: The Thermo microplate reader software was started, and the wavelength was set to 450 nm to 620 nm. The bottom of the microplate was wiped with absorbent paper, then the microplate was inserted into the slot of the microplate reader, and start was clicked for reading. The relative titer (potency) of the antibody was calculated based on the reading value.

### D. Antibody purification

D-1. The purification column was prepared and fixed on a foam float stand. The CNBr-Sepharose-activated resin was taken out from the 4°C refrigerator and equilibrated at room temperature for 0.5 h. Next, 0.3 g (to prepare 1 mL of the column) of the CNBr-Sepharose-activated resin was weighed and added to a 4 mL centrifuge tube, fully dissolved with pre-cooled 1 mM HCl (pH 3.0), then transferred to the purification column, washed with 50 mL of 1 mM HCl, and then soaked with 5 mL of 1 mM HCl (pH 3.0) for 10 min.

D-2. Subsequently, 5 mg of polypeptide was dissolved in 4 mL of binding buffer, and thorough mixing was allowed. A resulting solution was transferred to a 4 mL centrifuge tube, which was incubated for 2 h to 3 h at room temperature under repeated inverting.

D-3. The incubated filler was transferred to the purification column, and the flow-through fraction was collected. A resulting purification column was washed with 10 mL of blocking buffer and then soaked in the blocking buffer at room temperature for 2 h.

D-4. The resulting purification column was washed with a wash buffer 3 and a wash buffer 4 alternately, with 3 times by each wash buffer and 5 mL for each time.

D-5. If not used temporarily, the freshly-prepared purification column was washed with 10 mL of PBS, followed by 5 mL of 20% ethanol, and soaked in 20% ethanol at 4°C for storage.

D-6. If used immediately, the freshly-prepared purification column was washed with 20 mL of phosphate buffered saline (PBS). The pre-treated sample to be purified was circulated in the purification column.

D-7. After the sample was circulated for 45 min, the flow-through fraction was collected. The column filler was washed with 20 mL of PBS, followed by 10 mL of a wash buffer 2, and finally 10 mL of PBS.

D-8. Elution was conducted with the elution buffer, with the elution buffer added dropwise in 5 mL increments. The neutralization buffer was added to a 15 mL centrifuge tube for collection. After the elution was conducted with 20 mL of the elution buffer, the eluate was detected by Coomassie brilliant blue to determine whether the protein was eluted. If no protein was detected, the elution was completed. If protein was still detected, the elution was further conducted with 10 mL of the elution buffer. The detection was repeated until no protein was eluted. The purification filler was washed with 20 mL of the elution buffer, followed 10 mL of PBS, and then 10 mL of a storage buffer. The column was soaked in the storage buffer in a volume 3 times that of the column for storage at 4°C.

D-9. The antibody specificity was detected by the above ELISA method. The antibody concentration was measured by the bicinchoninic acid (BCA) assay.

### 1.4 Western blot assay

Protein extraction: The cell status was observed, and cells at logarithmic growth phase were collected and lysed. The original medium was removed, and the cells were washed twice with pre-cooled PBS, which was then discarded. The cells to be collected were placed on ice, an appropriate volume of radioimmunoprecipitation assay (RIPA) lysis buffer (a proteinase inhibitor, a phosphatase inhibitor, and phenylmethanesulfonyl fluoride (PMSF) were added in advance) was added according to the cell quantity for lysis. Cells in the culture dish were scraped off by a cell scraper, transferred by a pipette to a 1.5 mL EP tube. The solution was aspirated and dispensed 6 to 8 times with a 1 mL syringe until the cell lysate was no longer viscous. The mixture was allowed to stand on ice for 30 min to ensure complete lysis. The tube was centrifuged in a 4°C centrifuge at 12,000 rpm for 10 min to produce a supernatant, which was a protein product. The protein product was transferred to another 1.5 mL EP tube.

Determination of the protein concentration by the BCA method: Plotting of a standard curve: 0 µL, 1 µL, 2 µL, 4 µL, 8 µL, 12 µL, 16 µL, and 20 µL of protein standard (concentration: 0.5 µg/µL, BSA) were added to wells of a 96-well plate, and 20 µL, 19 µL, 18 µL, 16 µL, 12 µL, 8 µL, 4 µL, and 0 µL of cell lysis buffer were added respectively to make a total volume of 20 µL, such that protein standard concentrations in the wells were 0 µg/mL, 25 µg/mL, 50 µg/mL, 100 µg/mL, 200 µg/mL, 300 µg/mL, 400 µg/mL, and 500 µg/mL, respectively. Three replicate wells were set for each concentration. Afterwards, 1 µL of protein sample to be tested and 19 µL of protein lysis buffer were added to each well of the 96-well plate. Three replicate wells were set for each sample. Preparation of a BCA working solution: According to instructions, solutions A and B were mixed in a ratio of 50:1 to produce the BCA working solution, and 200 µL of the BCA working solution was added to each well. The above two 96-well plates each were incubated in a 37°C incubator for 30 min in the dark. It was guaranteed that there were no bubbles in each well. The absorbance value at a wavelength of 562 nm was read by a microplate reader. The standard curve was plotted based on the measured concentrations of the protein standard, and the concentration of each protein sample to be tested was calculated. The determined protein samples were adjusted with a 1 × loading buffer to the same concentration, subjected to thorough mixing, then placed in a metal bath and boiled at 100°C for 4 min to 8 min based on the amount of the sample, and subjected to long-term storage in a -80°C freezer.

Electrophoresis: The prepared gel was assembled in an electrophoresis tank, and electrophoresis buffer was added. Loading wells were flushed with a syringe to ensure that there were no impurities or bubbles in the loading wells. Protein samples and the marker were prepared to ensure a consistent loading volume in the loading wells. In a stacking gel, electrophoresis was conducted at a constant voltage of 80 V. In a separating gel, electrophoresis was conducted at a constant voltage of 120 V.

Membrane transfer: A 1 × transfer buffer was prepared in advance and pre-cooled in a 4°C cold room. The polyvinylidene fluoride (PVDF) membrane was placed in methanol for 15 s, then placed in ddH₂O for 2 min, and finally soaked in the transfer buffer. A transfer cassette, a sponge, and a sheet of filter paper were prepared, fully cleaned with ddH₂O, and soaked in the transfer buffer. The glass plate was taken out and rinsed with ddH₂O. The gel was taken out and soaked in the transfer buffer. The transfer cassette was arranged with a black side facing downwards, and then the sponge, filter paper, gel, PVDF membrane, filter paper, and sponge were stacked on the transfer cassette sequentially. Air bubbles were eliminated. The transfer cassette was closed and placed in a transfer tank, and a pre-cooled transfer buffer was poured into the transfer tank. According to the molecular weight of the target protein, the membrane transfer was conducted at a constant current of 200 mA for about 2 h.

Blocking: The transferred membrane was taken out from the transfer tank, placed in a prepared 5% skimmed milk powder solution, and slowly shaken on a horizontal shaker to allow blocking at room temperature for 2 h.

Antibody incubation: The blocked membrane was taken out from the blocking solution and washed twice with 1 × Tris buffered saline with Tween 20 (TBST). Primary antibody incubation: The washed membrane was completely immersed in a prepared primary antibody solution, slowly shaken on a horizontal shaker, incubated overnight at 4°C or for 2 h at room temperature, transferred to a membrane-washing box, rinsed twice with 1 × TBST, and then washed three times for 10 min each time. Secondary antibody incubation: A corresponding HRP-conjugated secondary antibody was selected based on the species of the primary antibody, and prepared into a secondary antibody solution at a ratio specified in instructions. The membrane incubated with the primary antibody was completely immersed in the secondary antibody solution, slowly incubated on a horizontal shaker at room temperature for 2 h, transferred to a membrane-washing box, rinsed twice with 1 × TBST, and then washed three times for 15 min/time.

Chemiluminescent detection with horseradish peroxidase (HRP): According to the manual of the chemiluminescent solution, solutions A and B were mixed in a ratio of 1:1 to prepare the chemiluminescent solution, which was stored in the dark. The membrane incubated with the secondary antibody was placed on a chemiluminescent plate, and an appropriate amount of the chemiluminescent solution was added dropwise. Exposure was allowed in a pre-cooled exposure device, and results were observed and analyzed.

### 1.5 Co-immunoprecipitation (Co-IP)

Preparation of cell lysate: The medium in the 10 cm culture dish was discarded, cells were washed once with pre-cooled 1 × PBS, and the PBS was removed. Next, 500 µL of pre-cooled 1× cell lysis buffer was added, and the culture dish was placed on ice for 5 min. The cells were scraped off from the culture dish by a cell scraper, transferred to a 1.5 mL EP tube, and placed on ice. A resulting cell lysate was drawn and injected by a 1 mL syringe repeatedly until the cell lysate was no longer viscous, and centrifuged in a 4°C centrifuge at 12,000 rpm for 10 min. The supernatant was transferred to another 1.5 mL EP tube, and the protein concentration was determined.

Pre-clearing for the cell lysate: 500 µg of cell lysate was added to 20 µL of 50% protein A/G agarose bead slurry. The mixture was incubated under rotating on a shaker at 4°C for 30 min, and then centrifuged in a 4°C centrifuge at 12,000 rpm for 10 min. The supernatant was transferred to a new 1.5 mL EP tube.

Immunoprecipitation: The primary antibody was added proportionally to the pre-treated cell lysate for incubation on a shaker at 4°C overnight. Then, 20 µL of 50% protein A/G agarose bead slurry was added, and incubation was conducted for 2 h on a shaker at 4°C. A resulting system was centrifuged in a 4°C centrifuge at 12,000 rpm for 30 s. A resulting supernatant was discarded, 500 µL of 1 × cell lysis buffer was added, and thorough mixing was conducted for washing. The above steps (3) and (4) were repeated three times.

Sample analysis: 20 µL to 40 µL of 2 × SDS lysis buffer was added to the precipitate above, and a resulting mixture was vortexted, and centrifuged for 30 s. The sample was placed in a 100°C metal bath and boiled for 2 min to 5 min, and instantaneously centrifuged at 12,000 rpm for 1 min. A resulting supernatant was collected and tested by SDS-PAGE.

### 1.6 Immunohistochemistry (IHC) analysis

In the present disclosure, a commercial ovarian cancer tissue microarray (purchased from Shanghai Outdo Biotech Co., Ltd.) was adopted. The ovarian cancer tissue microarray could be used directly according to the following steps:
Dewaxing: The glass slide was soaked in xylene - xylene - 100% alcohol - 100% alcohol - 95% alcohol - 90% alcohol - 80% alcohol - 70% alcohol sequentially. Xylene was the main reagent for dewaxing. Based on the like-dissovles-like rule, the glass slide was typically placed in each reagent for 10 min. In hot weather, the soaking time in each reagent could be shortened properly. In cold weather, the soaking time in each reagent could be appropriately extended to 12 min to 15 min.
Antigen retrieval: The dewaxed glass slide was rinsed with clear water for a specified period of time, and then soaked in 3% H₂O₂ for 10 min to remove the endogenous catalase. The H₂O₂ was poured off. The glass slide was washed twice with clear water, then placed in a citrate buffer, cooked in a microwave oven for 3 min (medium heat) under just boiling, cooled to room temperature, cooked once again, and cooled to room temperature. The cooking was intended to make antigen sites exposed.
Serum blocking: After the cooling was conducted to room temperature, the citrate buffer was poured off. The glass slide was washed twice with water, followed by PBS for 5 min each time, and wipe-dried to remove PBS around the tissue. Serum was immediately added to block some non-specific sites, and incubation was conducted in a 37°C incubator for 0.5 h. The serum was 10-fold diluted (a blocking solution included 900 µL of PBS and 100 µL of serum).
Addition of primary antibody: The blocked glass slide was taken out from the incubator and wipe-dried with absorbent paper to remove the serum around tissues on back and front sides of the glass slide, and then the primary antibody was added. If a control test was conducted, PBS was added to the control tissue. After the primary antibody was added, the glass slide was stored overnight in a 4°C refrigerator.
Addition of secondary antibody: The glass slide was taken out from the refrigerator, washed with PBS three times for 5 min each time, and wipe-dried to remove PBS around the tissue. The secondary antibody was then added, and incubation was conducted in a 37°C incubator for 0.5 h.
Addition of streptavidin-biotin complex (SABC): The incubated glass slide was taken out from the incubator, washed with PBS three times for 5 min each time, and wipe-dried to remove PBS around the tissue. The SABC was then added, and incubated in a 37°C incubator for 0.5 h. SABC was 100-fold diluted (990 µL of PBS : 10 µL of SABC).
Addition of chromogenic solution: The incubated glass slide was taken out from the incubator, washed with PBS three times for 5 min each time, and wipe-dried to remove PBS around the tissue. The chromogenic solution was added (preparation of the chromogenic solution: 1 drop of chromogenic reagent A was added to 1 mL of water, and thorough shaking was conducted. Then, 1 drop of chromogenic reagent B was added, and thorough shaking was conducted. One drop of chromogenic reagent C was added, and thorough shaking was conducted). A: DAB, B: H₂O₂, and C: phosphate buffer.
Counterstaining: The chromogenic glass slide was rinsed with clear water for a specified period of time, and then soaked in hematoxylin for staining. Generally, an animal tissue can be stained for 30 s, and a plant tissue can be stained for 3 min to 5 min.
Dehydration: The counterstained glass slide was rinsed with water and then soaked in 70% alcohol - 80% alcohol - 90% alcohol - 95% alcohol - 100% alcohol - 100% alcohol - xylene - xylene sequentially. The soaking in each reagent was conducted for 2 min. The soaking in xylene was conducted in a fume hood.
Mounting: The neutral balsam was dropped beside the tissue, and the glass slide was then covered with a cover slide. One side of the glass slide was laid flat, and then the other side of the glass slide was gently lowered, so as to avoid bubble generation. After the mounting was completed, the mounted glass slide was air-dried in a fume hood. The observation and photographing were conducted under a microscope.

### Example 2 Experimental results

### 2.1 Preliminary analysis of ABCG1 phosphorylation sites

ABCG1 (NCBI: NM_016818.3/NP_058198.2-ABCG1-666aa) expressed and purified in ovarian cancer cells was sent to APTBIO for mass spectrometry analysis of protein phosphorylation modifications. According to analysis results, six peptide fragments were identified. Among the six peptide fragments, four peptide fragments were preliminarily confirmed to have amino acid sites Y97, T99, S441, and Y655 as phosphorylation sites. For the remaining two peptide fragments, one peptide fragment has an amino acid site S120 or S125 phosphorylated, and it was also possible that both were phosphorylated simultaneously, as they were located accurately within an ATP-binding domain. In the other peptide fragment, T272 or S277 was phosphorylated or both were phosphorylated (as shown in FIG. 1). These results needed to be further verified through experiments. Secondary mass spectrometry spectra determined for the peptide fragments are shown in FIG. 2.

### 2.2 Detection of ABCG1 phosphorylation with antibodies

Pan-specific antibodies against phosphorylated serine, phosphorylated threonine, and phosphorylated tyrosine were used to detect purified ABCG1. It was found that these antibodies all could detect the specific bands corresponding to purified ABCG 1 (indicated by arrows in FIG. 3A). However, phosphorylated bands at the same positions could not be clearly detected in a lysate of GST-ABCG1-expressing cells. It was speculated that this may be attributed to a relatively low expression level of ABCG1, especially a relatively low phosphorylation level of serine, threonine, or tyrosine of ABCG1, in transiently-transfected cells (HEK293T), resulting in a small proportion in the total phosphorylated proteins in the cell lysate. When an equal amount of the sample was detected with the GST antibody, the level of GST-ABCG1 in the cell lysate was nearly 5 times to 10 times lower than that of purified GST-ABCG1 (the region without a band was selected as a background, and analysis was conducted by the ImageJ software) (as shown in FIG. 3B). In the pull-down assay using the anti-phosphorylated serine, threonine, or tyrosine antibody, followed by detection of the pull-down product with the GST antibody, it was found that the phosphorylation level of serine was the highest, while the phosphorylation level of tyrosine was the lowest (as shown in FIG. 3C). This indicated that phosphorylation levels in ABCG1 were ranked as follows: serine phosphorylation level > threonine phosphorylation level > tyrosine phosphorylation level. Of course, the influence of antibody affinity or specificity differences on these detection results could not be excluded. These data provide preliminary evidence that ABCG1 is likely phosphorylated at these amino acids..

### 2.3 Detection of ABCG1 phosphorylation sites in ovarian cancer cells and tissues

Eight specific anti-amino acid phosphorylation antibodies were prepared to detect ovarian cancer cell lines and ovarian cancer tissues (as shown in FIG. 4 to FIG. 5). It was confirmed that, in the ABCG1 protein, there were at least 8 amino acid sites that were phosphorylated, namely, Y97, T99, S120, S125, T272, S277, S441, and Y655.

The basic principles, major features, and advantages of the present disclosure are illustrated and described above. For those skilled in the art, it is obvious that the present disclosure is not limited to the details of the above exemplary embodiments, and the present disclosure can be implemented in other specific forms without departing from the spirit or basic features of the present disclosure. Accordingly, the embodiments should be regarded in all points of view as exemplary and not restrictive. The scope of the present disclosure is defined by the appended claims rather than the above description. Therefore, all changes falling within the meanings and scopes of equivalent elements of the claims should be included in the present disclosure. The reference numerals in the claims should not be considered as limiting the involved claims.

In addition, it should be understood that, although this specification is described by way of embodiments, not each embodiment only includes one independent technical solution, and this description mode in the specification is only for clarity. Those skilled in the art should take the specification as a whole. The technical solutions in the embodiments can also be properly combined to produce other embodiments that can be understood by those skilled in the art.

## Claims

1. Use of a reagent and/or device for detecting a human ABCG1 phosphorylation site in preparation of a reagent for risk assessment, diagnosis, or prognostic assessment of a tumor.

2. Use of a reagent for inhibiting a human ABCG1 phosphorylation site in preparation of a drug for preventing and/or treating a tumor, wherein preferably, the human ABCG1 phosphorylation site is one or more selected from the group consisting of Y97, T99, S120, S125, T272, S277, S441, and Y655.

3. The use according to claim 1 or 2, wherein the human ABCG1 phosphorylation site is one or more selected from the group consisting of Y97, T99, S120, S125, T272, S277, S441, and Y655.

4. The use according to any one of claims 1 to 3, wherein the tumor is selected from the group consisting of osteosarcoma, glioma, lung cancer, leukemia, and ovarian cancer.

5. A human ABCG1 protein phosphorylation-specific antigenic peptide, wherein the human ABCG1 protein phosphorylation-specific antigenic peptide is any one selected from the group consisting of amino acid sequences shown in SEQ ID NOs: 1-8.

6. A human ABCG1 protein phosphorylation site-specific antibody, wherein the human ABCG1 protein phosphorylation site-specific antibody is produced by immunizing an animal with the human ABCG1 protein phosphorylation-specific antigenic peptide according to claim 5.

7. Use of the human ABCG1 protein phosphorylation-specific antigenic peptide according to claim 5 in preparation of a formulation for detecting an ABCG1 protein phosphorylation site.

8. Use of the human ABCG1 protein phosphorylation site-specific antibody according to claim 6 in preparation of a formulation for detecting an ABCG1 protein phosphorylation site.

9. The use according to claim 7 or 8, wherein the ABCG1 protein phosphorylation site is one or more selected from the group consisting of Y97, T99, S120, S125, T272, S277, S441, and Y655.

10. A kit for risk assessment, diagnosis, prevention, treatment, or prognostic assessment of a tumor, comprising the reagent for detecting a human ABCG1 phosphorylation site according to claim 1, the reagent for inhibiting/promoting a human ABCG1 phosphorylation site according to claim 2, or the human ABCG1 protein phosphorylation site-specific antibody according to claim 6.
